# EUROPEAN PATENT SPECIFICATION

(11) **EP 2 979 574 B1**
(45) Date of publication and mention of the grant of the patent: **31.05.2017**
(21) Application number: 15178480.8
(22) Date of filing: 27.07.2015
(51) Int. Cl.: A45D 40/16, A61Q 1/02, B65B 1/24

(54) **METHOD TO PRODUCE A MAKE-UP COSMETIC PRODUCT**
VERFAHREN ZUR HERSTELLUNG EINES MAKE-UP-KOSMETIKPRODUKTS
PROCÉDÉ POUR PRODUIRE UN PRODUIT COSMÉTIQUE DE MAQUILLAGE

(30) Priority: 31.07.2014 IT MI20141407
(43) Date of publication of application: 03.02.2016
(73) Proprietor: Chromavis S.p.A., 20122 Milano (IT)
(72) Inventor: LARCERI, Nicolò, 20077 Melegnano (MI) (IT)
(74) Representative: Vanosi, Adelio Valeriano

(56) References cited:
- EP-A1- 1 871 335
- EP-A1- 2 204 106
- EP-A1- 2 220 959
- KR-A- 20080 050 755

## Description

The present invention relates to a production method cosmetic make-up product.

In particular, it relates to a method of making a cosmetic make-up product of the baked type, consisting of at least two make-up products with different features one from the other, such as different colors.

As known, the cosmetics industry is always looking for new methods for producing cosmetic products decorated in such a way as to be very appealing to the target audience.

Patent EP2218436B1, of the same applicant, describes a method of making a cosmetic make-up product implemented using a mold provided with reliefs, used along with a template in which the mold reliefs penetrate to make grooves inside a base cosmetic. Thereafter such grooves are filled with a further decorative cosmetic product using an additional template.

A cosmetic product is thus obtained which is decorated with high definition patterns and with clear and accurate lines and contours.

However, the working method is particularly laborious and difficult to be used on a large scale.

Patent application EP2220959 A1, again of the same applicant, describes a method for making a cosmetic make-up product which consists in depositing a base cosmetic paste on a bottom, pressing it with a first mold that has a smooth surface, spreading a cosmetic powder on the surface of the base cosmetic paste and pressing the whole with a second mold provided with reliefs.

The semi-finished product is then manually cleaned by rubbing the product onto suitable abrasive sponges. With this technique, the decorative powder is pressed by the second mold into the cosmetic paste, especially in those areas where the reliefs of the second mold are present.

Also this technique provides good results. However, the decorative powder that is pressed by the mold reliefs to create the grooves in the underlying base is distributed in such grooves in an uneven manner and the amount that at the end of the process remains into the same is not fully satisfactory.

The object of the present invention is to provide a method of making a cosmetic make-up product which overcomes the technical drawbacks of the prior art.

More in particular, an object of the present invention is to provide a method of making a cosmetic make-up product which can be applied on an industrial scale and which ensures a good reproducibility and productivity.

Another object of the present invention is to provide a method able to make a cosmetic make-up product with very deep decorations, saturated with decorative cosmetic product and with a well-defined design.

This and other objects are achieved by a method implemented according to the technical teachings of the appended claims.

Further features and advantages of the invention will become apparent from the description of a preferred but non-exclusive embodiment of the device, shown by way of a non-limiting example in the accompanying drawings, in which:
figures 1 to 8 schematically show some steps of the process according to the present description;
figure 9 shows a cosmetic make-up product made according to the method in figures 1-8; and
figures 10 and 11 show alternative embodiments of the cosmetic make-up product.

With reference to the above figures, a method of making a cosmetic make-up product is shown, indicated as a whole with reference numeral 1.

The first step of the method provides for dispensing a base cosmetic paste 2 on a support 3 which actually is the base (or bottom) for a cosmetic product.

The base cosmetic paste 2 may be dispensed by an automatic equipment; it preferably has a dynamic viscosity in the range between 800,000 and 1,500,000 cP at 25°C, so as to be rather dense.

The bottom or support 3 may be of any material and shape compatible with that of the molds that will be used later. In any case, it is preferable that the bottom, for a product like that shown, be made of terracotta and with a substantially circular shape.

The second step of the method provides for pressing the base cosmetic paste 2 using a first mold 4 heated at a temperature higher than 60 °C. The first mold is provided with a plurality of reliefs adapted to create grooves in the base cosmetic paste 2 during pressing.

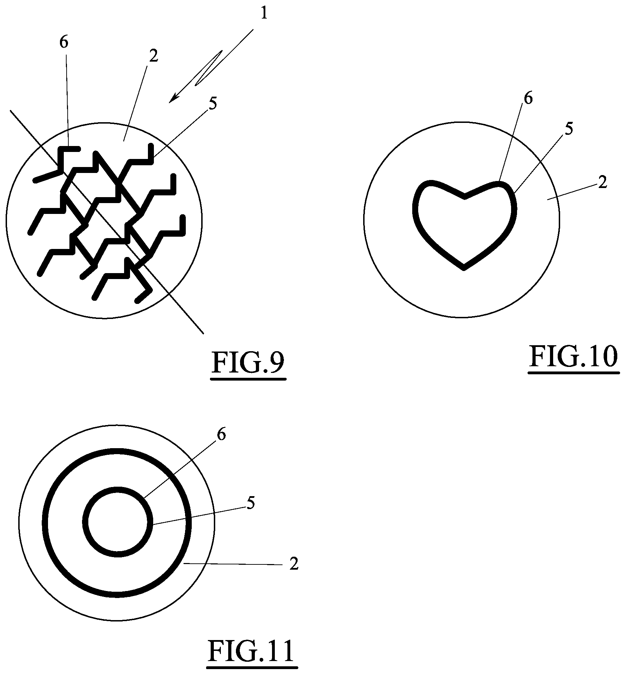

The reliefs may be arranged so as to create a pattern on the surface of the product, and in the case shown they are arranged so to represent a set of broken lines (see figure 9). Of course, they may have any shape or wording (such as a product brand). It must be said that the reliefs have a height h advantageously in the range between 1 and 4 mm, preferably 2.7 mm.

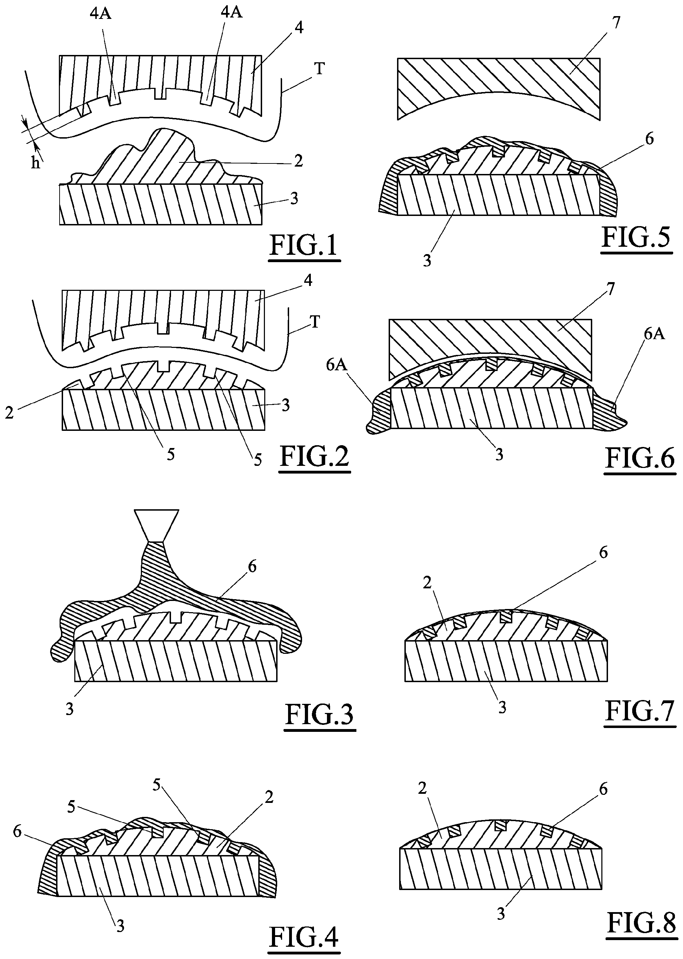

Figure 1 shows a step in which first mold 4 is about to close on the bottom to press the base cosmetic paste 2 just dispensed.

Figure 2 instead shows a step immediately following the opening of the first mold.

It has been verified that the use of a hot mold allows obtaining perfectly defined grooves in the base cosmetic paste 2 and that are able to have a much greater depth and definition compared to that obtained in a non-heated mold.

It is believed that this is due to the fact that hot mold makes the solvent present in the interface surface between the mold evaporate. The surface part therefore hardens immediately compared to the underlying one and thus is able to better retain the shape imposed by first mold 4, and specifically by the reliefs thereof. In fact, perfectly defined grooves 5 with very clear edges appear into base cosmetic paste 2.

Another hypothesis is that by at least partially eliminating the solvent (making it evaporate) in the interface surface between mold and base cosmetic paste 2, the hot mold promotes the detachment of the mold from the paste which actually occurs in a very sharp manner. In practice, the mold does not hold onto the base cosmetic paste 2 as it is lifted from the same, and this allows achieving excellent results in terms of definition and depth of the grooves.

Using a base cosmetic paste 2 in which the main solvent is water, it has been seen that it is preferable to use a temperature in the range between 90 °C and 120 °C, but even better if the temperature is between 95 °C and 105 °C. In this case, the optimal temperature is 100 °C, which corresponds to the vaporization temperature of water (i.e. the main solvent of the base cosmetic paste 2) at atmospheric pressure.

Therefore, it is deemed that the optimal temperature for the mold is the boiling temperature of the main solvent used in the base cosmetic paste 2.

Advantageously, a canvas is interposed between first mold 4 and the base cosmetic paste 2 before pressing the latter. The canvas is for example of the type produced by Nastri-tex company, of the soft type:
- composition: 80% polyamide/20% elastane
- surface density: 70 [g/m²]
- thickness: 100 micron

The interposed canvas has a double function of at least partially absorbing the solvent that is squeezed from the paste during pressing and preventing any residues of cosmetic paste from adhering to the mold surface or obstructing the grooves.

The third step of the method, shown in figure 3, provides for dispensing a second decorative cosmetic paste 6 on top of the base cosmetic paste 2 worked at the preceding step, so as to cover it completely and so as to fill at least partly the grooves 5 made in the base cosmetic paste 2 by the first hot mold 4.

To this end, the second decorative cosmetic paste 6 has a smoother texture than the base cosmetic paste 2, and in particular it has such a viscosity as to allow it to be regularly distributed in the grooves created by the pressure of the mold. For example, the viscosity is in the range between 300,000 and 500,000 cP, measured at 25 °C.

Figure 6 shows the step immediately following the pressing with a second mold 7 of the base cosmetic paste 2 and of the second decorative cosmetic paste 6 so as to make both take the shape of the second mold 7, thus obtaining a semi-finished product.

It is noted that the excess of second decorative cosmetic paste 6, in this step, is discharged from the edges of the mold, which advantageously may have openings or passages intended precisely for that purpose.

Advantageously, the surface of the second mold 7 intended to contact the cosmetic paste is smooth and without reliefs. It also has a convexity substantially corresponding to the final one of the cosmetic product.

Of course, it should be said that the surface of the second mold may also have other shapes, such as flat, wavy, with different convexity, depending on the final result to be achieved.

However, it is advantageous that the shape of the second mold, in that area intended to press the cosmetic paste, corresponds to that of the first hot mold, except for the reliefs. In fact, the rough shape of the base cosmetic product is defined by the first mold in those areas not affected by the reliefs.

The semi-finished product produced following the steps described above is then dried. In particular, the drying takes place in a furnace at a temperature in the range between 30° and 65 °C and for a time between 6 and 24 h.

In any case, irrespective of the technique used for drying, it is advantageous that it continues until a residual amount of solvent (that in the base and in the decorative paste, preferably water) of less than 1.5% by weight is contained in the semi-finished product.

The last step provides for the dried semi-finished product to be worked up to removing the decorative cosmetic paste at least from those areas of the base cosmetic paste free from grooves, so as to expose them.

Such a working may take place in a conventional manner by mechanical removal of at least one of the surface part of the base and decorative cosmetic paste, such as through rubbing with an abrasive sponge.

Alternatively, the surface working may be a turning process, which allows removing the part to be removed in a dry and firm manner, thus preventing the colors from blending together and thus improving still further the definition of the finished product.

In this way, the grooves filled with decorative paste are well defined on the surface of the cosmetic product for make-up, which make the cosmetic product very appealing.

The shape and the arrangement of the reliefs on the mold (and thus that of the grooves, and thus of the decorations) may be of any type. By way of example, figures 10 and 11 show alternative patterns that may be made on the final cosmetic product.

Various embodiments of the invention have been described but others may be conceived using the same innovative concept.

## Claims

1. A production method of a cosmetic make-up product (1) comprising the steps of:
a. dispensing a base cosmetic paste (2) on a support (3)
b. pressing the base cosmetic paste (2) through a first mold (4) heated at a temperature higher than 60 °C and provided with at least one relief (4A) adapted to create a groove (5) in the base cosmetic paste (2);
c. dispensing a second decorative cosmetic paste (6) over the base cosmetic paste (2) so as to completely cover it and so as to fill at least partially the groove (5) created in the base cosmetic paste (2) by the first mold (4);
d. pressing with a second mold (7) the base cosmetic paste (2) and the second decorative cosmetic paste (6) so as to make the pastes take a shape of the second mold (7), thus obtaining a semi-finished product;
e. drying the semi-finished product;
f. surface working the dried semi-finished product so as to remove the second decorative cosmetic paste (6) at least from those areas of the base cosmetic paste free from grooves (5).

2. A method according to the preceding claim, wherein the base cosmetic paste (2) has a dynamic viscosity in the range between 800,000 and 1,500,000 cP measured at 25 °C and/or wherein the decorative cosmetic (6) paste has a viscosity in the range between 300,000 and 500,000 cP measured at 25 °C.

3. A method according to one or more of the preceding claims, wherein the first mold (4) temperature is in the range between 90 °C and 120 °C, preferably between 95 °C and 105 °C but more preferably of 100 °C.

4. A method according to one or more of the preceding claims, wherein the height of the reliefs (4A) of the first mold is in the range between 1 and 4 mm, preferably 2.7 mm.

5. A method according to one or more of the preceding claims, wherein the pressing with the first heated mold (4) and/or with the second mold (7) takes place by interposing a canvas (T), preferably made of polyamide and elastane.

6. A method according to one or more of the preceding claims, wherein the second mold (7) is free from reliefs.

7. A method according to one or more of the preceding claims, wherein the drying takes place in a furnace at a temperature in the range between 30 and 65 °C and for a time between 6 and 24 h.

8. A method according to one or more of the preceding claims, wherein the base cosmetic paste (2) and decorative paste (6) comprise a volatile solvent and the drying lasts until the residual amount of volatile solvent in the semi-finished product is less than 1.5% by weight.

9. A method according to one or more of the preceding claims, wherein the surface working takes place by mechanical removal of the surface part at least one among the base cosmetic paste and the decorative cosmetic paste and/or wherein the surface working is a turning process.

## Patentansprüche

1. Herstellungsverfahren eines Kosmetik-Schminkprodukts (1), das die Schritte umfasst:
a. Spenden einer Basiskosmetikpaste (2) auf einen Träger (3)
b. Pressen der Basiskosmetikpaste (2) durch eine auf eine Temperatur höher als 60 °C erwärmte und mit mindestens einem Relief (4A) bereitgestellte erste Form (4), die angepasst wird, eine Vertiefung (5) in der Basiskosmetikpaste (2) zu erzeugen;
c. Spenden einer zweiten dekorativen Kosmetikpaste (6) über die Basiskosmetikpaste (2), um sie so vollständig abzudecken und um so mindestens teilweise die in der Basiskosmetikpaste (2) durch die erste Form (4) gebildete Vertiefung (5) auszufüllen;
d. Pressen der Basiskosmetikpaste (2) und der zweiten dekorativen Kosmetikpaste (6) mit einer zweiten Form (7), damit die Pasten eine Gestalt der zweiten Form (7) annehmen, um somit ein Halbfertigprodukt zu erhalten;
e. Trocknen des Halbfertigprodukts;
f. Oberflächenbehandlung des getrockneten Halbfertigprodukts, um so die zweite dekorative Kosmetikpaste (6) mindestens von jenen Bereichen der Basiskosmetikpaste zu entfernen, die frei von Vertiefungen (5) sind.

2. Verfahren nach dem vorhergehenden Anspruch, wobei die Basiskosmetikpaste (2) eine dynamische Viskosität in dem Bereich zwischen 800.000 und 1.500.000 cP gemessen bei 25 °C hat und/oder wobei die dekorative Kosmetikpaste (6) eine Viskosität in dem Bereich zwischen 300.000 und 500.000 cP gemessen bei 25 °C hat.

3. Verfahren nach einem der vorhergehenden Ansprüche, wobei die erste Temperatur der Form (4) im Bereich zwischen 90 °C und 120 °C, vorzugsweise zwischen 95 °C und 105 °C aber besonders bevorzugt bei 100 °C liegt.

4. Verfahren nach einem der vorhergehenden Ansprüche, wobei die Höhe des Reliefs (4A) der ersten Form in dem Bereich zwischen 1 und 4 mm, vorzugsweise 2,7 mm ist.

5. Verfahren nach einem der vorhergehenden Ansprüche, wobei das Pressen mit der ersten erwärmten Form (4) und/oder mit der zweiten Form (7) durch Zwischenschalten eines vorzugsweise aus Polyamid und Elastan hergestellten Gewebes (T) stattfindet.

6. Verfahren nach einem der vorhergehenden Ansprüche, wobei die zweite Form (7) frei von Reliefs ist.

7. Verfahren nach einem der vorhergehenden Ansprüche, wobei das Trocknen in einem Ofen bei einer Temperatur im Bereich zwischen 30 und 65 °C und für eine Zeit zwischen 6 und 24 Stunden stattfindet.

8. Verfahren nach einem der vorhergehenden Ansprüche, wobei die Basiskosmetikpaste (2) und dekorative Paste (6) ein flüchtiges Lösungsmittel umfassen und das Trocknen dauert, bis die restliche Menge an flüchtigem Lösungsmittel in dem Halbfertigprodukt geringer als 1,5 Gew.-% ist.

9. Verfahren nach einem der vorhergehenden Ansprüche, wobei die Oberflächenbehandlung durch mechanisches Entfernen des Oberflächenteils mindestens eines unter der Basiskosmetikpaste und der dekorativen Kosmetikpaste stattfindet und/oder wobei die Oberflächenbehandlung ein Drehprozess ist.

## Revendications

1. Méthode de production d'un produit cosmétique de maquillage (1) comprenant les étapes suivantes :
a. distribution d'une pâte cosmétique de base (2) sur un support (3) ;
b. passage sous pression de la pâte cosmétique de base (2) dans un premier moule (4) chauffé à une température supérieure à 60°C, et doté d'au moins un évidement (4A) adapté pour créer un sillon (5) dans la pâte cosmétique de base (2) ;
c. distribution d'une deuxième pâte cosmétique décorative (6) sur la pâte cosmétique de base (2) de façon à la recouvrir entièrement, en remplissant ce faisant au moins partiellement le sillon (5) créé dans la pâte cosmétique de base (2) par le premier moule (4) ;
d. passage sous pression de la pâte cosmétique de base (2) et de la deuxième pâte cosmétique décorative (6) dans un deuxième moule (7) de façon que les pâtes adoptent une forme du deuxième moule (7), en obtenant ainsi un produit semi-fini ;
e. séchage du produit semi-fini ;
f. travail de la surface du produit semi-fini séché de façon à enlever la deuxième pâte cosmétique décorative (6) au moins des zones de la pâte cosmétique de base exemptes de sillons (5).

2. Méthode selon la revendication précédente, la pâte cosmétique de base (2) présentant une viscosité dynamique dans la plage comprise entre 800 000 et 1 500 000 cP mesurée à 25°C et/ou la pâte cosmétique décorative (6) présentant une viscosité comprise entre 300 000 et 500 000 cP mesurée à 25°C.

3. Méthode selon une ou plusieurs des revendications précédentes, la température du premier moule (4) étant comprise dans la plage 90°C à 120°C, de préférence entre 95°C à 105°C, mieux encore de 100°C.

4. Méthode selon une ou plusieurs des revendications précédentes, la hauteur des évidements (4A) du premier moule étant comprise entre 1 et 4 mm, et de préférence égale à 2,7 mm.

5. Méthode selon une ou plusieurs des revendications précédentes, la pression avec le premier moule chauffé (4) et/ou avec le deuxième moule (7) étant effectuée par l'interposition d'une toile (T) de préférence en polyamide et en élastane.

6. Méthode selon une ou plusieurs des revendications précédentes, le deuxième moule (7) étant dépourvu d'évidements.

7. Méthode selon une ou plusieurs des revendications précédentes, le séchage étant effectué dans une étuve à une température comprise entre 30 et 65°C, et, pendant une période comprise entre 6 et 24 h.

8. Méthode selon une ou plusieurs des revendications précédentes, la pâte cosmétique de base (2) et la pâte cosmétique décorative (6) comprenant un solvant volatil, et le séchage durant jusqu'à ce que la quantité résiduelle de solvant volatil dans le produit semi-fini soit inférieure à 1,5 % en poids.

9. Méthode selon une ou plusieurs des revendications précédentes, le travail de la surface s'effectuant par l'extraction mécanique de la partie de la surface d'au moins une des pâtes que sont la pâte cosmétique de base et la pâte cosmétique décorative, et/ou le travail de la surface étant une opération de travail au tour.
